(19) Europäisches Patentamt
European Patent Office
Office européen des brevets

(11) Publication number: **0 082 658**
**A2**

(12) # EUROPEAN PATENT APPLICATION

(21) Application number: 82306650.1

(22) Date of filing: 13.12.82

(51) Int. Cl.³: C 07 D 471/04
C 07 C 127/19
//A61K31/44, (C07D471/04, 221/00, 209/00)

(30) Priority: 17.12.81 US 331494
24.12.81 US 334195
30.09.82 US 425151
30.09.82 US 425152

(43) Date of publication of application: 29.06.83 Bulletin 83/26

(84) Designated Contracting States: AT BE CH DE FR GB IT LI LU NL SE

(71) Applicant: PFIZER INC.
235 East 42nd Street
New York, N.Y. 10017(US)

(72) Inventor: Massett, Stephen Sargent
78 Brandegee Avenue
Groton, New London Connecticut(US)

(72) Inventor: Welch, Willard McKowan, Jr.
166 Pequot Avenue
Mystic, New London Connecticut(US)

(72) Inventor: Watson, Harry Austin, Jr.
694 Eastern Point Road
Groton, New London Connecticut(US)

(74) Representative: Wood, David John et al,
Pfizer Limited Ramsgate Road
Sandwich Kent CT13 9NJ(GB)

(54) **Processes and intermediates useful in the preparation of flutroline.**

(57) 1,1-Di(*p*-fluorophenyl)urea, 2-carbobenzoxy-8-fluoro-5-(*p*-fluorophenyl)-2,3,4,5-tetrahydro-1H-pyrido[4,3-b]indole, an efficient process for converting the former to the latter, further comprising conversion of the latter to 8-fluoro-5-(*p*-fluorophenyl)-2-[4-(*p*-fluorophenyl)-4-hydroxybutyl)]-2,3,4,5-tetrahydro-1H-pyrido[4,3-b]indole (flutroline) or to 8-fluoro-5-(*p*-fluorophenyl)-2,3,4,5-tetrahydro-1H-pyrido[4,3-b]indole (an alternative flutroline intermediate); and further, novel processes and intermediates used in converting the latter to flutroline.

EP 0 082 658 A2

# PROCESSES AND INTERMEDIATES USEFUL IN THE PREPARATION OF FLUTROLINE

This invention relates to novel intermediates and processes useful in the preparation of 8-fluoro-5-(p-fluorophenyl)-2-[4-(p-fluorophenyl)-4-hydroxybutyl)]-2,3,4,5-tetrahydro-1H-pyrido[4,3-b]indole, also known as flutroline, a neuroleptic agent having valuable therapeutic activity, see Plattner *et al.*, U.S. Patent No. 4,001,263, and Harbert *et al.*, J. Med. Chem. 23, pp. 635-643.

Plattner *et al.* and Harbert *et al.* also describe the earliest synthesis of flutroline: p-Fluorophenylhydrazine is condensed with N-carbethoxy-4-piperidone to form 8-fluoro-2-carbethoxy-2,3,4,5-tetrahydro-1H-pyrido[4,3-b]indole. By the Ullman reaction the latter is arylated to form the 5-(p-fluorophenyl) derivative and then hydrolyzed under vigorous basic conditions to yield 8-fluoro-5-(p-fluorophenyl)-2,3,4,5-tetrahydro-1H-pyrido[4,3-b]indole. The synthesis of flutroline is completed by 2-alkylation with 3-(p-fluorobenzoyl)propyl chloride and finally reduction of the ketone group to an alcohol group.

Welch, U.S. Patent No. 4,014,890 has described an alternative synthesis of the same earlier intermediate 8-fluoro-5-(p-fluorophenyl)-2-carbethoxy-2,3,4,5-tetrahydro-1H-pyrido[4,3-b]indole, *viz.*, by condensation of 1,1-di(p-fluorophenyl)hydrazine with N-carbethoxy-4-piperidone. In contrast to the present 2-carbobenzoxy intermediate, the earlier 2-carbethoxypyridoindole is a waxy solid difficult to isolate and purify by crystallization. Furthermore, in the further conversion to flutroline, the carbethoxy

group must be removed under harsh, basic conditions, producing undesirable by-products which interfere with further processing (particularly by hydrogenation) and render difficult the isolation of flutroline in the highly purified form required for its use in therapy.

Welch, U.S. Patent No. 4,267,331 describes the preparation of related hexahydropyridoindole by reductive alkylation of 8-fluoro-5-(p-fluorophenyl)-2,3,4,4a,5,9b-hexahydro-1H-pyrido[4,3-b]indole. Surprisingly, the present reductive alkylations, reduction of amide side chain and reduction of alkyne side chain proceed without concomitant reduction of the 4a,9b-double bond a benzylic hydroxyl.

Murakami et al., Heterocycles 12, pp. 1571-1574 (1979) have described the preparation of unsymmetrical diarylhydrazines by reaction of aqueous sodium hypochlorite with unsymmetrical diarylureas in ethanol. Such hydrazines were condensed with pyruvate to form N-arylindole derivatives. In the present instance, Murkami's conditions failed to provide the required 1,1-di(p-fluorophenyl)hydrazine.

The present 2,3,4,5-tetrahydro-1H-pyrido[4,3-b]-indoles are alternatively named as 1,2,3,4-tetrahydro-gamma-carbolines. In either case, the ring system is numbered as in the formula

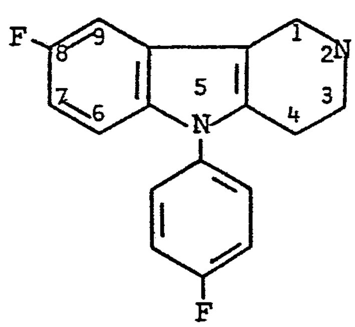

which defines the radical R as used hereinafter.

The present invention embraces a process for preparing 2-carbobenzoxy-8-fluoro-5-(p-fluorophenyl)-2,3,4,5-tetrahydro-1H-pyrido[4,3-b]indole, which comprises the steps of

(a) chlorinating 1,1-di(p-fluorophenyl)urea with a chlorinating agent, such as a $(C_1-C_4)$alkyl hypochlorite, in a step (a) reaction-inert solvent to form a first intermediate, 3-chloro-1,1-di(p-fluorophenyl)urea, *in situ*;

(b) rearranging the intermediate 3-chlorourea by the action of an alkali metal $(C_1-C_3)$alkoxide in a step (b) reaction-inert solvent to form a second intermediate, 2-$(C_1-C_3)$carbalkoxy-1,1-di(p-fluorophenyl)hydrazine, *in situ*;

(c) hydrolyzing and decarboxylating the intermediate carbalkoxyhydrazine with water in the presence of base in a step (c) reaction-inert solvent to form a third intermediate, 1,1-di(p-fluorophenyl)hydrazine, *in situ*;

(d) condensing the intermediate 1,1-di(p-fluorophenyl)hydrazine with N-carbobenzoxy-4-piperidone in the presence of a strong acid in a step (d) reaction-inert solvent, and recovering said 2-carbobenzoxy-8-fluoro-5-(p-fluorophenyl)-2,3,4,5-tetrahydro-1H-pyrido[4,3-b]indole.

The preferred $(C_1-C_4)$alkyl hypochlorite is t-butyl hypochlorite. The preferred alkali metal alkoxide is sodium methoxide, whereby the second intermediate is specifically 2-carbomethoxy-1,1-(p-diphenyl)hydrazine. The preferred strong acid is a mineral acid. Most preferred is hydrochloric acid.

The above process further comprises hydrogenation of said 2-carbobenzoxy-8-fluoro-5-(p-fluorophenyl)-2,3,4,5-tetrahydro-1H-pyrido[4,3-b]indole over a noble metal catalyst to form 8-fluoro-5-(p-fluorophenyl)-2,3,4,5-tetrahydro-1H-pyrido[4,3-b]indole (R-H, an alternative intermediate also useful in the preparation of flutroline); or a similar such hydrogenation but in the presence of substantially one molar equivalent of a lactol, 2-(p-fluorophenyl)-5-hydroxytetrahydrofuran, to form flutroline. In either case, the preferred noble metal is palladium and the preferred hydrogen pressure is 2-8 atmospheres.

Also embraced by the present invention is the separate process step for the conversion of 8-fluoro-5-(p-fluorophenyl)-2-carbobenzoxy-2,3,4,5-tetrahydro-1H-pyrido[4,3-b]indole to flutroline per se, as delineated above; and the present novel and valuable flutroline intermediates 1,1-di(p-fluorophenyl)urea and 2-carbobenzoxy-8-fluoro-5-(p-fluorophenyl)-2,3,4,5-tetrahydro-1H-pyrido[4,3-b]indole.

The present invention further comprises the preparation of flutroline by contacting R-H (as defined above) with at least an equimolar quantity of 2-(p-fluorophenyl)-5-hydroxytetrahydrofuran in a reaction inert solvent. Preferred reducing agents are (1) alkali cyanoborohydrides, preferably sodium cyanoborohydride and (2) hydrogen, at a pressure of about 1-3 atmospheres and a catalytically effective amount of a noble metal catalyst such as palladium or platinum, preferably palladium at a temperature from about -10°C. to 50°C. Preferred solvents for this reaction include $(C_1-C_3)$-lower alkyl acetates, for example ethyl acetate.

The present invention also comprises the preparation of flutroline by the two step sequence of first condensing R-H in a reaction inert polar organic

solvent with at least an equimolar amount of an acetylenic compound of the formula

$$HC\equiv CCHOH(\underline{p}F_6H_4)$$

and (with respect to said acetylenic compound) an equimolar amount of formaldehyde, in the presence of a catalytically-effective amount of cuprous chloride or cuprous bromide; and thereafter hydrogenating the resulting condensation product, of the formula

$$R\text{-}CH_2\text{-}C\equiv C\text{-}CHOH(\underline{p}FC_6H_4) \quad ,$$

(V)

in a reaction inert solvent at about 15-75°C. and a hydrogen pressure of up to about 75 psig in the presence of a catalytically-effective amount of a noble metal catalyst.

The present invention also encompasses the novel intermediate, 8-fluoro-5-(p-fluorophenyl)-2-[4-(p-fluorophenyl)-4-hydroxy-2-butynyl]-2,3,4,5-tetrahydro-1H-pyrido[4,3-b]indole.

The present invention provides a further convenient method for preparing flutroline from the intermediate R-H (wherein R is as defined above) and readily available reagents, via a novel, readily purified intermediate. This intermediate is particularly well-suited for the preparation of flutroline in the state of purity required for its use as a therapeutic agent.

The reactions and intermediate involved in the present invention are schematically represented as follows:

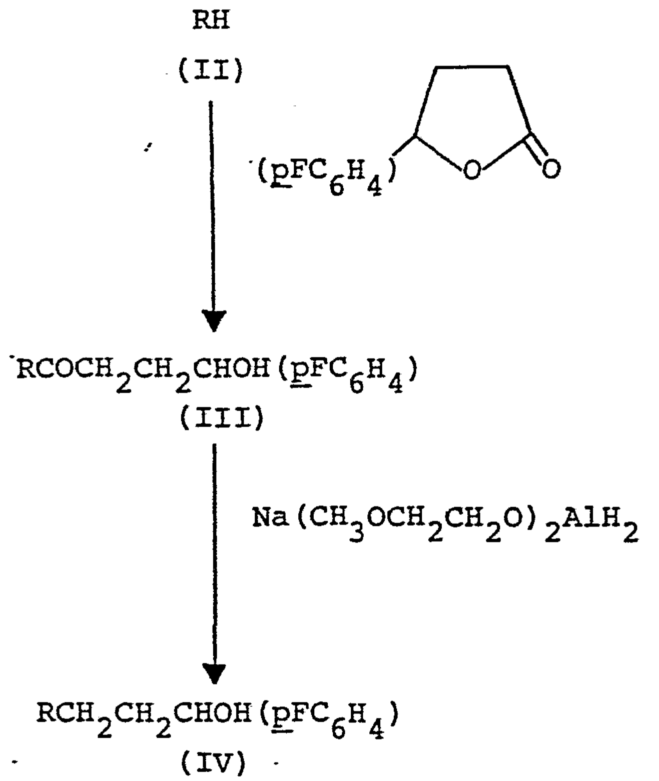


RH

(II)

$RCOCH_2CH_2CHOH(\underline{p}FC_6H_4)$

(III)

$RCH_2CH_2CHOH(\underline{p}FC_6H_4)$

(IV)

wherein R is as defined above.

A salient feature of the present invention is a process for flutroline (IV) which comprises reduction of 8-fluoro-5-(p-fluorophenyl)-2-[4-(p-fluorophenyl)-4-hydroxybutyryl]-2,3,4,5-tetrahydro-1H-pyrido[4,3-b]-indole (III) with sodium bis-(2-methoxyethoxy)aluminum hydride at 80-110°C in a reaction inert solvent. It is surprising that this hydride reducing agent will selectively reduce the carbonyl of the primary amide group to methylene, without concomitant, interfering reduction of the 4a,9b-double bond of the group R to 4a,9b-dihydro and/or of the side chain benzylic hydroxyl group to hydrogen.

The above process further comprises the preparation of the compound (III) by the reaction of R-H (II) with 5-(p-fluorophenyl)tetrahydro-2-furanone at 65-125°C in a reaction inert solvent.

5-(p-Fluorophenyl)tetrahydro-2-furanone is alternatively named as a 5-substituted dihydro-2(3H)-furanone; as a 4-substituted 4-butanolide; or as a 4-substituted 4-hydroxybutyrolactone.

As used herein, the expression "reaction-inert solvent" refers to a solvent which does not interact with reagents, intermediates or products in such a manner as to adversely affect conversion to the desired intermediate, or the yield or quality of the desired product. In all cases, suitable reaction-inert solvents are disclosed and exemplified below.

The present flutroline intermediate, 2-carbobenzoxy-8-fluoro-5-(p-fluorophenyl)-2,3,4,5-tetrahydro-1H-pyrido[4,3-b]indole is prepared by the four steps, (a) to (d), which are defined above. The various step intermediates can be isolated, if desired. However, it is one of the features of the present highly efficient process to simply employ these intermediates *in situ*, avoiding the time consuming isolation and the inevitable loss of these valuable intermediates, while at the same time attaining the above flutroline intermediate in a high state of purity, suitable for further processing (particularly processing which involves hydrogenation) and ultimately yielding flutroline in the high state of purity required for its use in therapy.

Step (a) of the present process, chlorination of 1,1-di(p-fluorophenyl)urea, can be carried out by the action of substantially one equivalent of a $(C_1-C_4)$-alkyl hypochlorite or a similar chlorinating agent. For reasons of mutual solubility in a common solvent, an alkyl hypochlorite is preferred; for reasons of ready availability and solubility properties, t-butyl hypochlorite is most preferred. Lower temperatures

are generally used for the chlorination step, e.g., -10 to 10°C., conveniently 0-5°C. obtained by an ice-water bath. The preferred reaction-inert solvents are water-misible, readily dissolve both reactants, and are suitable for the further steps (b) to (d). Especially well-suited are ($C_1$-$C_3$)alkanols, particularly methanol.

Step (b) involves rearrangement with loss of the elements of HCl and gain of the elements of ($C_1$-$C_3$)-alkanol to form the intermediate carbalkoxy hydrazine. This step is readily accomplished by simple addition of substantially two equivalents of an alkali metal ($C_1$-$C_3$)alkoxide, conveniently sodium methoxide, maintaining the same reaction inert-solvent as used in step (a). The alkoxide is conveniently added at the temperature used for step (a). Step (b) proceeds readily at moderate temperatures, e.g., 0-50°C., and is conveniently carried out at ambient temperatures, usually in the range of about 17-27°C.

Step (c), which involves base catalyzed hydrolysis and decarboxylation, is readily accomplished by simply adding excess water to the reaction mixture, otherwise maintaining the same reaction-inert solvent as used in steps (a) and (b). Step (c) occurs most readily at somewhat higher temperature than steps (a) and (b), e.g., 50-100°C., preferably about 60-75°C. When the solvent boils below the desired reaction temperature, the solvent is partially removed by distillation, optionally with the addition of more water. Alternatively, the reaction is carried out under pressure.

Step (d), formation of the desired carbobenzoxy-pyridoindole from the step (c) hydrazine and N-carbobenzoxy-4-piperidone involves initial formation of a hydrazone, which is followed by cyclization. If

desired, the initial phase is accomplished simply by adding the piperidine and excess of a $(C_1-C_4)$alkanoic acid, preferably and conveniently acetic acid, to the hydrazine product of step (c), and warming to about 60-110°C., conveniently to reflux (about 65 to 100°C., depending upon the exact composition of solvents in the reaction mixture). Cyclization is then accomplished by adding an excess of a strong acid, preferably a strong mineral acid such as conc. hydrochloric acid, followed by further heating in the same elevated temperature range. Alternatively and preferably, both hydrazone formation and cyclization are accomplished by heating in the strong acid, omitting the separate step in weak acid. The intermediate product, 2-carbobenzoxy-8-fluoro-5-(p-fluorophenyl)-2,3,4,5-tetrahydro-1H-pyrido[4,3-b]indole, is readily recovered by standard methods of solvent displacement, extraction, concentration and crystallization.

Generally, the above intermediate product is further processed by a hydrogenation step, either hydrogenation to 8-fluoro-5-(p-fluorophenyl)-2,3,4,5-tetrahydro-1H-pyrido[4,3-b]indole (which can then be converted to flutroline by one of a number of methods, e.g., as further disclosed herein or according to references cited above), or hydrogenation in the presence of substantially one equivalent of the lactol, 2-(p-fluorophenyl)-5-hydroxytetrahydrofuran, producing flutroline.

In either case, the hydrogenation is carried out in a reaction-inert solvent, under a hydrogen atmosphere, in the presence of a hydrogenation catalyst such as nickel, or a noble metal. Such catalysts are used in an amount sufficient to catalyze the process. As is well known in the art of catalytic hydrogenation, the amount of catalyst required will vary with the

nature of the catalyst, the activity of a particular batch of catalyst and the exact conditions of the hydrogenation (e.g., the reactor, the type and amount of agitation, temperature, pressure, solvent). While the amount of catalyst will be generally similar to the amounts which are illustrated in the examples below, routine experimentation will define optimal amounts in specific cases.

The noble metal catalysts as employed in the present invention include platinum, palladium, rhenium, rhodium and ruthenium, either of the supported or non-supported type, as well as the known catalytic compounds thereof such as the oxides, chlorides, etc. Examples of suitable catalyst supports include carbon, silica and barium sulfate. The catalysts may be preformed or formed in situ by reduction of an appropriate salt of the catalytic compound. Examples of preferred catalysts are 5% palladium-on-carbon, 5% platinum-on-carbon, 5% rhodium-on-carbon, platinum chloride, palladium chloride, platinum oxide and ruthenium oxide. The most highly preferred catalyst for reasons of economy and efficiency, is palladium, particularly palladium supported on carbon.

The pressure of the present hydrogenation is not critical and can range from subatmospheric to 100 atmospheres, or higher. Moderate pressures of about 2-8 atmospheres are preferred, since the hydrogenation generally proceeds at a reasonable rate at these pressures and the elaborate and expensive equipment required for higher pressure hydrogenation is avoided.

The temperature of the hydrogenation is also not critical. Temperatures ranging from 0-75°C. are generally satisfactory. As a matter of convenience, ambient temperatures (about 17-27°C.) are usually preferred, the cost of cooling or heating thus being avoided.

For the present hydrogenation processes, examples of suitable solvents are ($C_1$-$C_3$)alkyl acetates; ($C_1$-$C_4$)alkanols; ethers such as dimethoxyethane, tetrahydrofuran, dioxane; or isopropyl ether; glycol or glycol monoethers such as 2-methoxyethanol; hydrocarbons such as benzene, toluene and xylene; halocarbons such as methylene chloride or chloroform; water; or combinations of two or more of these solvents. The preferred solvent is either ethyl acetate, or a mixture of ethyl acetate and a ($C_1$-$C_3$)alkanol.

Flutroline (per se or as a pharmaceutically-acceptable salt thereof), as well as the present alternative flutroline intermediate also derived by hydrogenation, are recovered by standard methods of solvent displacement, extraction, concentration and crystallization which are well known in the art.

The required 1,1-di(p-fluorophenyl)urea is conveniently prepared from di(p-fluorophenyl)amine by the action of sodium cyanate in the presence of trifluoroacetic acid, following the method of Murakami (supra). The di(p-fluorophenyl)amine is conveniently prepared by an improved Ullman method, see J. Org. Chem. 26, p. 2721 (1961). The required N-carbobenzoxy-4-piperidone is readily available by reaction of carbobenzyoxy chloride with 4-piperidone under conditions well known in the art, or from N-benzyl-4-piperidone as specifically described below. Finally, preparation of the requisite lactol is according to methods disclosed in U.S. Patent No. 4,267,331 (supra).

Flutroline is optionally formed from the compound R-H under typical reductive alkylation conditions. Methods for carrying out such reactions have been reviewed, for example, by Emerson, Organic Reactions 4, 174 (1948) and by Rylander in "Catalytic Hydrogenation Over Platinum Metals", Academic Press,

New York, 1967, p. 291-303. The reaction may be effected with a wide variety of reducing agents known to be useful for reductive alkylation of secondary amines with aldehydes and ketones such as, for example, hydrogen in the presence of a catalytic amount of a noble metal catalyst such as platinum, palladium, rhodium, ruthenium or nickel; various metal hydride reducing agents such as sodium cyanoborohydride, sodium borohydride and lithium borohydride; and formic acid. Preferred reducing agents are (1) sodium cyanoborohydride or (2) hydrogen in the presence of noble metal catalysts. Especially preferred noble metals are platinum and palladium and most particularly preferred is palladium for reasons of economy and efficiency.

Preferably, the amine (R-H) is contacted with an equimolar amount of the lactol, 2-(p-fluorophenyl)-5-hydroxytetrahydrofuran and one of the above-mentioned preferred reducing agents in the presence of a reaction inert organic solvent at a temperature of from about -10° to 50°C. When the preferred reducing agent is sodium cyanoborohydride, at least an equivalent amount is employed. When the preferred noble metal catalysts are employed, the reaction is carried out in the presence of a molar excess of hydrogen.

As mentioned above, the noble metal catalyst is employed in a "catalytic amount", which term is well understood by those skilled in the art. When the noble metal catalysts and hydrogen are employed, the reaction may be carried out at atmospheric pressure or at high pressures up to about 3 atmospheres or higher with equal facility. The factor which will ordinarily determine whether the reaction is carried out at atmospheric pressure or higher pressure is the scale on which reaction is carried out. For example,

when carried out with a few grams or less of reactants, atmospheric pressure is more convenient; however, on a commercial scale, use of higher pressure is usually preferable.

Examples of suitable reaction inert solvents are lower alkyl acetates of from 1 to 3 carbon atoms, preferably ethyl acetate, lower alkanols, such as methanol, ethanol, isopropanol and n-butanol, ethers such as dimethoxyethane, diethyleneglycol dimethyl ether, ethyl ether and isopropyl ether, glycols such as ethylene glycol and diethylene glycol, and glycol monoethers such as 2-methoxyethanol and diethyleneglycol monomethyl ether.

While reaction can be carried out at temperatures of from about -50° up to the reflux temperature of the solvent, preferred reaction temperature is from about -10° to 50°C. for reasons of convenience and efficiency. The product is then isolated by standard methods and purified, if desired, for example, by crystallization or chromatography.

In accordance with a further process of this invention, substantially equimolar amounts of the unsubstituted amine starting material (R-H), the acetylenic reagent and formaldehyde are employed to effect the condensation reaction, which normally takes place within a period of about one to about 24 hours. The reaction is normally carried out in a reactioninert polar organic solvent such as a water-miscible lower alkanol like methanol, ethanol or isopropanol, etc., or a cyclic ether of the class including dioxane and tetrahydrofuran, etc. In practice, mixtures of the two type solvents are usually employed. Any convenient source of formaldehyde, such as paraformaldehyde, may be used for

the reaction but it is preferable, in practice, to employ the 37% aqueous formaldehyde of commerce, which is commonly known as formalin. However, formaldehyde may also be generated in the reaction mixture *in situ* by first depolymerizing the readily available paraformaldehyde with concentrated hydrochloric acid. Upon completion of the reaction, the desired 2-unsaturated intermediate is readily recovered from the reaction mixture by first removing the solvents therefrom *via* evaporation under reduced pressure and then dissolving the resultant residue in a halogenated hydrocarbon solvent such as methylene chloride, followed by washing with dilute acid and base and then subjecting the dried organic extract to evaporation under reduced pressure. In this way, a residual product is obtained that can easily be triturated with a suitable solvent such as acetone and further purified, if necessary, by means of recrystallization in the usual manner from an appropriate solvent system.

The resulting acetylenic condensation product (V) is then subjected to catalytic hydrogenation, preferably accomplished by employing a noble metal catalyst such as palladium, usually suspended on a proper catalyst support such as carbon or barium sulfate, and the like. The preferred solvent for this reaction is the same as that employed in the condensation step, *viz*., a lower alkanol such as methanol or a cyclic ether such as tetrahydrofuran or mixtures thereof. Upon completion of the reduction step, the catalyst is easily separated from the reaction mixture by filtration and the solvent thereafter removed from the resulting filtrate by evaporation under reduced pressure. In this way, a crude residual

product is obtained that can then be easily subjected to such standard purification techniques as dissolution in hot ethyl acetate, followed by the addition of n-hexane, etc. so as to afford the desired final product (<u>viz</u>., flutroline in substantially pure form.

The required acetylenic reagents are known compounds which are easily synthesized by treating the appropriate benzaldehyde compound with acetylene in the presence of n-butyl lithium, according to the general procedure described by M. M. Midland in the Journal of Organic Chemistry, Vol. 40, No. 15, p. 2250 (1975), to yield the desired 1-hydroxy-1-phenyl-2-propyne.

The further transformation and intermediates involved in the processes of the present invention are schematically represented and summarized above, i.e. (II) ⟶ (III) ⟶ (IV).

For the sodium <u>bis</u>(2-methoxyethoxy)aluminum hydride reduction, aromatic hydrocarbons (such as benzene, toluene, xylenes, chlorobenzene) are well-suited as reaction inert solvents. In fact this hydride is commercially available in such a solvent. The preferred solvent is toluene or a benzene/toluene mixture. The reduction is carried out at elevated temperature (e.g. 80-110°), conveniently at the temperature of a steam bath. At least 3 equivalents of the hydride (1.5 mole/mole) are required. However, the reaction is not adversely affected by use of an excess of hydride (e.g. 4 equivalents). Indeed, use of some excess is helpful in forcing the reaction to completion in a reasonable period of time. The present selective reduction of amide, while maintaining the 4a,9b-double bond and benzylic hydroxyl is particularly surprising in view of the fact that such an excess of the hydride reducing agent is usually employed.

For the reaction of R-H (II) with 5-(p-fluorophenyl)tetrahydro-2-furanone, the former is simply warmed with the latter. At least one molar equivalent of the furanone is used, but usually it is more convenient to use an excess in order to force complete conversion of the more valuable R-H in a reasonable time period. Suitable solvents include aromatic hydrocarbons, chlorinated hydrocarbons and ethers as defined above. Again, the preferred solvent is toluene.

8-Fluoro-5-(p-fluorophenyl)-2,3,4,5-tetrahydro-1H-pyrido[4,3-b]indole (R-H, II) required for the above process variations is available by a number of methods (supra), preferably by the method exemplified below.

5-(p-Fluorophenyl)-2-furanone is readily prepared by acid catalyzed lactone formation from 4-(p-fluorophenyl)-4-hydroxybutyric acid. The latter is conveniently prepared by sodium borohydride reduction of 3-(p-fluorobenzoyl)propionic acid, in turn conveniently derived from fluorobenzene and succinic anhydride by the Friedel-Crafts reaction.

The present invention is illustrated by the following Examples. However, it should be understood that the invention is not limited to the specific details of these Examples.

0082658

PREPARATION 1

N-Carbobenzoxy-4-piperidone

N-Benzyl-4-piperidone (122.2 g, 0.645 mole) in 500 ml toluene was warmed to 45°C. Benzyl chloroformate (130 ml, 155 g, 0.915 mole) was added in a thin, steady stream, and the reaction mixture heated to reflux for 2 hours, cooled to ambient temperature, diluted with 250 ml $H_2O$ and stirred vigorously 0.5 hour. The organic layer was separated, washed 1 x 400 ml 6N HCl and then 1 x 100 ml saturated NaCl, dried ($MgSO_4$), treated with activated carbon, and concentrated to an oil. The oil was distributed between 400 ml ethyl acetate and 400 ml of $H_2O$ containing 67.0 g $NaHSO_3$, stirred 0.5 hour, and the aqueous layer separated, washed with 3 portions of ether, made basic with aqueous NaOH and extracted with fresh ether. The ether layer was dried and reevaporated to yield purified title product as an oil, 136.1 g.

## PREPARATION 2

## 3-(p-Fluorobenzoyl)propionic Acid

A suspension of 170 g anhydrous aluminum chloride and 266 g fluorobenzene was stirred under nitrogen at 10°C while succinic anhydride (54.4 g) was added in small portions. The mixture was stirred 1.5 hours at 10 to 20°C and then heated on a steam bath for 45 minutes. The mixture was poured onto 1.2 kg crushed ice containing 280 ml 6N HCl. The product was extracted into methylene chloride, which was washed with water, and then back-extracted into 2N NaOH. The basic extract was treated with activated carbon, and acidified with HCl to precipitate title product, 94 g, mp 98.5-101°C. Recrystallization from methylene chloride/hexane gave 90.6% recovery, mp 99-101°C.

## PREPARATION 3

## 5-(p-Fluorophenyl)tetrahydro-2-furanone

To the basic extract of the preceding Preparation was added 0.55 molar equivalent of sodium borohydride portionwise over 45 minutes during which the temperature rose to 41°C. The reaction mixture was allowed to cool to room temperature over 3 hours and then acidified with concentrated HCl. Intermediate 4-(p-fluorophenyl)-4-hydroxybutyric acid was extracted into methylene chloride. One-half volume of 6N hydrochloric acid was added to the organic extract and the mixture stirred at room temperature for 2 hours. The organic layer was separated and vacuum distilled to yield title product as a waxy, low melting solid. Yield: 82%, bp 120°/0.05 mm.

PREPARATION 4

A 200 ml. volume of dry tetrahydrofuran was cooled to -60°C in a dry ice/acetone bath and then acetylene gas was passed into the solvent until 8.0 g (0.307 mole) had dissolved. The resulting solution was then added to a second 200 ml. portion of dry tetrahydrofuran cooled to -70°C. in a flame-dried 1-liter three-necked, round bottomed reaction flask equipped with magnetic stirrer, addition funnel and nitrogen inlet tube to provide a dry nitrogen atmosphere. To this solution, there was then slowly added 99 ml. of a 2.22 molar solution of n-butyl lithium (0.22 mole) at such a rate that the internal temperature remained below -65°C. After the addition was complete, a solution consisting of 24.82 g (0.20 mole) of p-fluorobenzaldehyde dissolved in 80 ml. of tetrahydrofuran was added at such a rate that the internal temperature again remained below -65°C. The reaction mixture was then stirred at -72°C. for a period of 30 minutes and then allowed to warm up to +5°C. during another 30 minute period. The reaction was then quenched with 80 ml. of water, followed by the addition of anhydrous potassium carbonate to form a pasty mass and an organic supernatant liquid. The organic layer was decanted and combined with two subsequent ether washes of the potassium carbonate mass. The resulting orange solution was then dried over fresh anhydrous potassium carbonate for a period of approximately 16 hours (overnight) and subsequently distilled *in vacuo* to give 20.5 g (68%) of pure 1-hydroxy-1-(p-fluorophenyl)-2-propyne, b.p. 98-101°C./ 8 mm. Hg. A second preparation gave a 92% yield of pure product (b.p. 104°C./9 mm. Hg).

Anal. Calcd. for $C_9H_7FO$: C, 71.98; H, 4.70; F, 12.65.

Found: C, 71.71; H, 4.99; F, 12.70.

EXAMPLE 1

1,1-Di(p-fluorophenyl)urea

Di(p-fluorophenyl)amine (102.5 g., 0.5 mole) and NaOCN (65 g, 1.0 mole) were stirred in 700 ml $CH_2Cl_2$ at 16°C. $F_3CCOOH$ (84.5 ml, 117 g, 1.025 mole) was added as a thin stream over 5 minutes, during which the reaction mixture exothermed to 28°C. The exotherm peaked at 32°C. shortly after addition was complete. The reaction mixture was stirred at 23-25° for 21 hours, diluted with 350 ml $H_2O$ and stirred 0.5 hour. The organic layer was separated and stirred 0.25 hour with 40 g NaOH in 500 ml $H_2O$. The layers were separated, and the aqueous layer back washed with 100 ml $CH_2Cl_2$. The combined organic layer and back wash was dried ($MgSO_4$), concentrated to 600 ml, diluted with 600 ml isopropanol, reconcentrated to 600 ml, rediluted with 600 ml of fresh isopropanol, reconcentrated to 800 ml, rediluted with 400 ml fresh isopropanol and again reconcentrated to 800 ml. The mixture was cooled to room temperature (product began to crystallize) and then to 0-5°C. Title product was recovered by filtration, 104.9 g, m.p. 150-153°C.

EXAMPLE 2

Step (a) 1,1-Di(p-fluorophenyl)-3-chlorourea

t-Butyl hypochlorite (7.5 g, 0.070 mole) was added to 1,1-di(p-fluorophenyl)urea (17.5 g, 0.071 mole) in 440 ml methanol at 0-5° and the mixture stirred for 1 hour at that temperature to produce a solution containing step (a) title product.

Step (b) 1,1-Di(p-fluorophenyl)-2-carbomethoxyhydrazine

At 0-5°C, sodium methoxide (7.55 g, 0.14 mole) in 100 ml of methanol was added to the solution of step (a) product. The mixture was stirred 0.5 hour at 0-5°C and 2 hours at ambient temperature to yield a solution of step (b) title product.

Step (c) 1,1-Di(p-fluorophenyl)hydrazine

Water (100 ml) was added to the solution of step (b) product and methanol removed by distillation until the pot temperature was 74°C. The mixture was then refluxed 16 hours and finally cooled to ambient temperature to yield a solution of step (c) title product.

Step (d) 8-Fluoro-5-(p-fluorophenyl)-2-carbobenzoxy-2,3,4,5-tetrahydro-1H-pyrido-[4,3-b]indole

The solution from step (c) was diluted with 35 ml acetic acid and 16.2 grams (0.0695 mole) of N-carbobenzoxy-4-piperidone was added. The reaction mixture was heated to reflux for 30 minutes, cooled to ambient temperature, acidified with 60 ml conc. HCl, and refluxed an additional 1.5 hours. After

EXAMPLE 2 (Cont.)

adding 100 ml of water, the solution was refluxed for 5 minutes and filtered hot. The solids were washed with water at 45°C., followed by 50 ml of cold methanol, and then dissolved in 400 ml $CH_2Cl_2$. The resulting solution was dried ($MgSO_4$), treated with activated charcoal and filtered. By repeated addition of methanol and distillation to a final pot temperature of 65°C. and a final volume of 200 ml, $CH_2Cl_2$ was displaced with methanol. The resulting slurry of step (d) title product was recovered by filtration, 17.8 g, m.p. 157-160°C.

Anal. Calcd for $C_{25}H_{20}N_2O_2F_2$:

C, 71.76; H, 4.82; N, 6.70; m/e 418

Found: C, 71.76; H, 4.91; N, 6.69; m/e 418

## EXAMPLE 3

### Step (a) 1,1-Di(*p*-fluorophenyl)-3-chlorourea

*t*-Butyl hypochlorite (22.8 g, 0.21 mole) was added to 1,1-di(*p*-fluorophenyl)urea (49.4 g, 0.2 mole) in 750 ml methanol at 0-5° and the mixture stirred for 0.5 hour to produce a thin slurry containing step (a) title product.

### Step (b) 1,1-Di(p-fluorophenyl)-2-carbomethoxy-hydrazine

At 0-5°C., sodium methoxide (22.7 g) in 250 ml methanol was added in a thin stream over 5 minutes to the step (a) product mixture. The reaction mixture was warmed to 40-45°C. for 15 minutes to produce a milky solution of step (b) title product.

### Step (c) 1,1-Di(*p*-fluorophenyl)hydrazine

NaOH (40 g) in 175 ml $H_2O$ was added to the step (b) product mixture. The mixture was distilled at ambient pressure to remove the methanol and the aqueous residue refluxed for 25 hours and cooled to ambient temperature to yield a solution of step (c) title product.

### Step (d) 8-Fluoro-5-(*p*-fluorophenyl)-2-carbobenzoxy-2,3,4,5-tetrahydro-1H-pyrido[4,3-b]indole

The solution from step (c) was diluted with 300 ml conc. HCl, maintaining 35°C. or less. N-Carbobenzoxy-4-piperidone (46.6 g, 0.2 mole) was added and the mixture heated to reflux for 1 hour. The resulting thick slurry was filtered hot with water and methanol wash, 64.6 g. The solids were taken into 400 ml $CHCl_2$, carbon treated, dried ($MgSO_4$), and the

EXAMPLE 3 (Cont.)

$CH_2Cl_2$ displaced with methanol to a final volume of 500 ml and purified step (d) title product recovered by filtration, 60.9 g, identical with title product of Example 2.

EXAMPLE 4

8-Fluoro-5-(p-fluorophenyl)-2-[4-(p-fluorophenyl)-4-hydroxybutyl]-2,3,4,5-tetrahydro-1H-pyrido[4,3-b]indole (Flutroline)

In a Parr bottle were combined 5% Pd/C (5 g of 50% water-wet), 8-fluoro-5-(p-fluorophenyl)-2-carbobenzoxy-2,3,4,5-tetrahydro-1H-pyrido[4,3-b]indole (5.0 g, 0.0119 mole) in 100 ml ethyl acetate, and 5.5 grams (0.03 mole) 5-(p-fluorophenyl)-2-hydroxytetrahydrofuran in 100 ml ethyl acetate. The mixture was agitated and hydrogenated at 30-40 psig for 20 hours. The solution was filtered to recover catalyst, with ethyl acetate and $CH_2Cl_2$ wash. The filtrate and washes were concentrated *in vacuo* to a viscous oil. The oil was dissolved in 400 ml ethyl acetate and filtered. The solution was concentrated to an oil, diluted with 150 ml ether, and filtered to yield title product, 3.8 gm, m.p. 145-149°C.

EXAMPLE 5

8-Fluoro-5-(p-fluorophenyl)-2,3,4,5-tetrahydro-1H-pyrido[4,3-b]indole

In a Parr bottle were combined 5% Pd/C (10 g of 50% water-wet) and 60 g of 8-fluoro-5-(p-fluorophenyl)-2-carbobenzoxy-2,3,4,5-tetrahydro-1H-pyrido[4,3-b]-indole (60 g, 0.143 mole) in 400 ml ethyl acetate and 100 ml methanol and the mixture hydrogenated at 44-80 psig for 4 hours. Catalyst was recovered by filtration and the filtrate evaporated to solids *in vacuo*. The residue taken into $CH_2Cl_2$, carbon treated, dried ($Na_2SO_4$) and $CH_2Cl_2$ displaced with hexane by distillation to a final pot temperature of 70°C. The white, crystalline product was recovered by filtration, 35.6 g, m.p. 126-129°C.

## EXAMPLE 6

### Flutroline

6.42 g (20.0 mmol) 8-Fluoro-5-(p-fluorophenyl)-2,3,4,5-tetrahydro-1H-pyrido[4,3-b]indole hydrochloride prepared according to U.S. Patent No. 4,001,263 (supra) was added portion wise to a rapidly stirred mixture of 109 ml of ethyl acetate, 27 ml of saturated sodium bicarbonate solution, and 27 ml water. A clear two phase system resulted after vigorous stirring at room temperature for about 30 minutes. The layers were separated and wet ethyl acetate layer was added to about 25 ml of an ethyl acetate solution containing about 30 mmol of 2-(p-fluorophenyl)-5-hydroxy-tetrahydrofuran. The solution of reactants was charged to a pressure bottle, 1.74 g of 5% palladium-carbon (50% water wet) was added and the mixture was hydrogenated at 30 psi in a Parr apparatus for 71 hours. Total hydrogen uptake was about 22 psi. The catalyst was filtered, repulped in 50 ml ethyl acetate, and refiltered. After drying over magnesium sulfate, the reaction solution was concentrated at reduced pressure to about 50 ml, 200 ml hexane was added and the solution was vacuum concentrated once again to about 50 ml. The light tan slurry was granulated in an ice bath for 30 minutes, filtered, and washed with cold hexane to yield 8.03 g (82%) of flutroline, m.p., 143.5 to 145.5°C.

## EXAMPLE 7

## Flutroline Hydrochloride Hydrate

A mixture of 333 mg (1.82 mmol) of 2-(*p*-fluorophenyl)-5-hydroxytetrahydrofuran and 500 mg (1.75 mmol) of 8-fluoro-5-(*p*-fluorophenyl)-2,3,4,5-tetrahydro-1H-pyrido[4,3-b]indole in 20 ml of methanol in a 100 ml 3-necked round bottom flask with magnetic stirrer, dropping funnel and $N_2$ atmosphere was stirred 15 minutes at room temperature and then a solution of 110 mg (1.75 mmol) of sodium cyanoborohydride in 5 ml of methanol was added dropwise over 20 minutes. The reaction mixture was then stirred 3 hours at room temperature at which time thin layer chromatography (4:1 ethyl acetate/methanol) showed the reaction to be essentially complete. The solvent was evaporated under reduced pressure and the residues were partitioned between ether and aqueous sodium hydroxide solution. The organic phase was separated, combined with one further ether wash to the aqueous phase, dried over magnesium sulfate and treated with a solution of hydrochloride gas in dry ether to give the title compound, 600 mg (76% yield), m.p. 233-235°C.

| | | |
|---|---|---|
| Anal. Calcd.: | C, 64.22; H, 5.34; N, 5.54. |
| Found: | C, 64.48; H, 5.27; N, 5.78. |

## EXAMPLE 8

### 8-Fluoro-5-(p-fluorophenyl)-2-[4(p-fluorophenyl)-4-hydroxy-2-butynyl]-2,3,4,5-tetrahydro-1H-pyrido[4,3-b]indole [R-$CH_2C{\equiv}CCHOH(pF_6H_4)$].

A solution consisting of 9.47 g. (0.033 mole) of 8-fluoro-5-(p-fluorophenyl)-2,3,4,5-tetrahydro-1H-pyrido[4,3-b]indole dissolved in 150 ml. of absolute ethanol was placed in a 500 ml. reaction flask under a dry nitrogen atmosphere and warmed to about 35°C., and then treated with 2.78 ml. of a 37% aqueous formaldehyde solution (0.033 mole) and 1.64 g. of cuprous chloride. As soon as the reaction mixture became homogeneous (a period of 1-2 minutes), a solution consisting of 5.0 g. (0.033 mole) of 1-hydroxy-1-(p-fluorophenyl)-2-propyne dissolved in 80 ml of dioxane was added. The resulting mixture was then stirred overnight at ambient temperature for a period of approximately 16 hours. At the end of this time, the solvents were removed in vacuo and the residues subsequently dissolved in 300 ml. of methylene chloride. After washing with dilute hydrochloric acid and dilute ammonium hydroxide, the organic phase was dried over anhydrous magnesium sulfate and filtered. The resulting filtrate was then evaporated to near dryness while under reduced pressure and the residue thus obtained was subsequently treated with 100 ml. of acetone to form a slurry. The pale yellow solid which resulted was thereafter recovered by means of suction filtration and subsequently recrystallized from 400 ml. of acetone and 100 ml. of methanol to give 5.8 g. (39%) of pure title product, m.p. 194-196°C.

Anal. Calcd. for $C_{27}H_{21}F_3N_2O$:
C, 72.63; H, 4.72; N, 6.27.
Found: C, 72.33; H, 4.94; N, 6.39.

EXAMPLE 9

Flutroline

A solution consisting of 100 mg (0.000245 mole) of title product of the preceding Example dissolved in 10 ml. of dry tetrahydrofuran was treated with 100 mg. of 5% palladium on barium sulfate catalyst and stirred in a hydrogen atmosphere for a period of 90 minutes. At the end of this time, the catalyst was separated from the reaction mixture by means of filtration and the solvent thereafter removed by means of evaporation under reduced pressure to afford a crude residual product. The latter material was then dissolved in 1.0 ml. of hot ethyl acetate and the resulting solution subsequently diluted with 15 ml. of n-hexane to give, upon cooling, 65 mg. (65%) of flutroline in the form of a crystalline deposit. The pure product melted at 143-144°C. and was identical in every respect with an authentic sample prepared according to the procedure described in U.S. Patent No. 4,001,263, as shown by thin layer chromatography.

## EXAMPLE 10

### 8-Fluoro-5-(p-fluorophenyl)-2-[4-(p-fluorophenyl)-4-hydroxybutyryl]-2,3,4,5-tetrahydro-1H-pyrido[4,3-b]indole [$R-COCH_2CH_2CHOH(p-FC_6H_4)$].

A mixture of 8-fluoro-5-(p-fluorophenyl)-2,3,4,5-tetrahydro-1H-pyrido[4,3-b]indole (0.01 mole) and 5-(p-fluorophenyl)tetrahydro-2-furanone (2.0 g) in toluene was heated on a steam bath overnight. An additional 1.0 g 5-(p-fluorophenyl)tetrahydro-2-furanone was added and the solution was stirred another 5 hours on the steam bath. The reaction mixture was cooled and poured into a stirred mixture of 100 ml water and 100 ml ethyl acetate. Precipitated solids were removed by filtration and the ethyl acetate layer was separated and concentrated *in vacuo* to an oil. The oil was chromatographed on a short silica gel column, with ethyl acetate as eluant and tlc monitoring. Clean product fractions were combined and evaporated to yield purified title product as an oil, 2.7 g; $R_f$ 0.43 on silica gel tlc with 9:1 $CHCl_3$: methanol as eluant; pnmr/$CDCl_3$/TMS includes delta 4.4 ppm for -OH; ir (KBr) 1626 $cm^{-1}$.

EXAMPLE 11

Flutroline [R-$CH_2CH_2CH_2CHOH$(p-$FC_6H_4$)]

A solution of 1.16 g 8-fluoro-5-(p-fluorophenyl)-2-[4-(p-fluorophenyl)-4-hydroxybutyryl]-2,3,4,5-tetrahydro-1H-pyrido[4,3-b]indole (0.0025 mole) in toluene (35 ml) was stirred under nitrogen while 0.0132 mole of sodium bis(2-methoxyethoxy)aluminum hydride (70% in benzene) was added. The resulting mixture was heated on a steam bath one hour, cooled to room temperature and poured into water, and extracted with ethyl acetate. The organic layer was concentrated *in vacuo*. The resulting residue was chromatographed on silica gel with ethyl acetate as eluant and monitoring by tlc (two solvent systems: 9:1 $CHCl_3$:methanol and acetonitrile) with authentic flutroline as control. Product fractions were combined, evaporated to dryness, the residue triturated with diisopropyl ether to yield title product, 175 mg; m.p. 141.5-144; mixed mp with authentic flutroline, no depression.

CLAIMS

1. A process for preparing 2-carbobenzoxy-8-fluoro-5-(p-fluorophenyl)-2,3,4,5-tetrahydro-1H-pyrido[4,3-b]indole, which comprises the steps of

(a) chlorinating 1,1-di(p-fluorophenyl)urea with a $(C_1-C_4)$alkyl hypochlorite in a step (a) reaction-inert solvent to form a first intermediate, 3-chloro-1,1-di(p-fluorophenyl)urea, *in situ*;

(b) rearranging the intermediate 3-chlorourea by the action of an alkali metal $(C_1-C_3)$alkoxide in a step (b) reaction-inert solvent to form a second intermediate, 2-$(C_1-C_3)$carbalkoxy-1,1-di(p-fluorophenyl)hydrazine, *in situ*;

(c) hydrolyzing and decarboxylating the intermediate carbalkoxyhydrazine with water in the presence of base in a step (c) reaction-inert solvent to form a third intermediate, 1,1-di(p-fluorophenyl)hydrazine, *in situ*;

(d) condensing the intermediate 1,1-di(p-fluorophenyl)hydrazine with N-carbobenzoxy-4-piperidone in the presence of a strong acid in a step (d) reaction-inert solvent, and recovering said 2-carbobenzoxy-8-fluoro-5-(p-fluorophenyl)-2,3,4,5-tetrahydro-1H-pyrido[4,3-b]indole.

2. A process of claim 1 wherein the $(C_1-C_4)$alkyl hypochlorite is t-butyl hypochlorite, the alkali metal alkoxide is sodium methoxide and the second intermediate is 2-carbomethoxy-1,1-(p-diphenyl)hydrazine, and the strong acid is hydrochloric acid.

3. A process of claim 1 or 2 which further comprises hydrogenation of said 2-carbobenzoxy-8-fluoro-5-(p-fluorophenyl)-2,3,4,5-tetrahydro-1H-pyrido[4,3-b]-indole over a noble metal catalyst in the presence of substantially one molar equivalent of 2-(p-fluorophenyl)-5-hydroxytetrahydrofuran, at 0-75°C. in a reaction-inert solvent, to form flutroline.

4. A process of claim 3 wherein the noble metal is palladium and the hydrogen pressure is 2 to 8 atmospheres.

5. A process of claim 1 or 2 which further comprises hydrogenation of said 2-carbobenzoxy-8-fluoro-5-(p-fluorophenyl)-2,3,4,5-tetrahydro-1H-pyrido[4,3-b]indole over a noble metal catalyst at 0-75°C. to form 8-fluoro-5-(p-fluorophenyl)-2,3,4,5-tetrahydro-1H-pyrido[4,3-b]indole.

6. A process of claim 5 wherein the noble metal is palladium and the pressure of hydrogen is 2-8 atmospheres.

7. A process of claim 5 or 6 which further comprises the preparation of flutroline by contacting said 8-fluoro-5-(p-fluorophenyl)-2,3,4,5-tetrahydro-1H-pyrido[4,3-b]indole with at least an equimolar quantity of 2-(p-fluorophenyl)-5-hydroxytetrahydrofuran in a reaction inert solvent in the presence of a reducing agent.

8. A process of claim 7 wherein said reducing agent is sodium cyanoborohydride or hydrogen and a catalytically effective amount of a noble metal catalyst.

9. A process of claim 5 or 6 which further comprises the preparation of flutroline by condensing said 8-fluoro-5-(p-fluorophenyl)-2,3,4,5-tetrahydro-1H-pyrido[4,3-b]indole in a reaction-inert polar organic solvent with at least an equimolar amount of an acetylenic compound of the formula

$$HC{\equiv}CCHOH(p\text{-}FC_6H_4)$$

and an equimolar amount of formaldehyde, with respect to said acetylenic compound, in the presence of a catalytically effective amount of cuprous chloride

or cuprous bromide; and thereafter hydrogenating the resulting condensation product in a reaction-inert organic solvent at about 15-75°C. and at a hydrogen pressure up to about 75 psig in the presence of a catalytically effective amount of a noble metal catalyst.

10. A process of claim 5 or 6 which further comprises the preparation of the 8-fluoro-5-(p-fluorophenyl)-2-[4-(p-fluorophenyl)-4-hydroxybutyryl]-2,3,4,5-tetrahydro-1H-pyrido[4,3-b]indole by reaction of said 8-fluoro-5-(p-fluorophenyl)-2,3,4,5-tetrahydro-1H-pyrido[4,3-b]indole with 4-(p-fluorophenyl)-tetrahydro-2-furanone at 65-125°C. in a reaction inert solvent.

11. A process of claim 10 which further comprises the preparation of flutroline by reduction of said 8-fluoro-5-(p-fluorophenyl)-2-[4-(p-fluorophenyl)-4-hydroxybutyryl]-2,3,4,5-tetrahydro-1H-pyrido[4,3-b]-indole with sodium bis-(2-methoxyethoxy)aluminum hydride at 80-110°C in a reaction-inert solvent.

12. A process for preparing flutroline which comprises hydrogenation of 2-carbobenzoxy-8-fluoro-5-(p-fluorophenyl)-2,3,4,5-tetrahydro-1H-pyrido[4,3-b]-indole over a noble metal catalyst in the presence of substantially one molar equivalent of 2-(p-fluorophenyl)-5-hydroxytetrahydrofuran, at 0-75°C. in a reaction-inert solvent.

13. A process of claim 12 where the noble metal is palladium and the hydrogen pressure is 2-8 atmospheres.

14. A process for the preparation of flutroline which comprises contacting 8-fluoro-5-(p-fluorophenyl)-2,3,4,5-tetrahydro-1H-pyrido[4,3-b]indole with at least an equimolar quantity of 2-(p-fluorophenyl)-5-hydroxytetrahydrofuran in a reaction inert solvent in the presence of a reducing agent.

15. A process of claim 14 wherein said reducing agent is sodium cyanoborohydride or hydrogen and a catalytically effective amount of a noble metal catalyst.

16. A process for the preparation of flutroline which comprises condensing 8-fluoro-5-(p-fluorophenyl)-2,3,4,5-tetrahydro-1H-pyrido[4,3-b]indole in a reaction inert polar organic solvent with at least an equimolar amount of an acetylenic compound of the formula

$$HC{\equiv}CCHOH(pFC_6H_4)$$

and an equimolar amount of formaldehyde, with respect to said acetylenic compound, in the presence of a catalytically effective amount of cuprous chloride or cuprous bromide; and thereafter hydrogenating the resulting condensation product in a reaction inert organic solvent at about 15-75°C. and at a hydrogen pressure up to about 75 psig in the presence of a catalytically effective amount of a noble metal catalyst.

17. A process for the preparation of flutroline which comprises the reduction of 8-fluoro-5-(p-fluorophenyl)-2-[4-(p-fluorophenyl)-4-hydroxybutyryl]-2,3,4,5-tetrahydro-1H-pyrido[4,3-b]indole with sodium bis-(2-methoxyethoxy)aluminum hydride at 80-110°C in a reaction-inert solvent.

18. A process of claim 17 which further comprises the preparation of the 8-fluoro-5-(p-fluorophenyl)-2-[4-(p-fluorophenyl)-4-hydroxybutyryl]-2,3,4,5-tetrahydro-1H-pyrido[4,3-b]indole by reaction of 8-fluoro-5-(p-fluorophenyl)-2,3,4,5-tetrahydro-1H-pyrido[4,3-b]indole with 4-(p-fluorophenyl)tetrahydro-2-furanone at 65-125°C in a reaction inert solvent.

19. 1,1-Di(p-fluorophenyl)urea.

20. 2-Carbobenzoxy-8-fluoro-5-(p-fluorophenyl)-2,3,4,5-tetrahydro-1H-pyrido[4,3-b]indole.

21. 8-Fluoro-5-(p-fluorophenyl)-2-[4-(p-fluorophenyl)-4-hydroxy-2-butynyl]-2,3,4,5-tetrahydro-1H-pyrido[4,3-b]indole.

22. 8-Fluoro-5-(p-fluorophenyl)-2-[4-(p-fluorophenyl)-4-hydroxybutyryl]-2,3,4,5-tetrahydro-1H-pyrido[4,3-b]indole.

CLAIMS FOR AUSTRIA

1. A process for preparing 2-carbobenzoxy-8-fluoro-5-(p-fluorophenyl)-2,3,4,5-tetrahydro-1H-pyrido[4,3-b]indole, which comprises the steps of

(a) chlorinating 1,1-di(p-fluorophenyl)urea with a $(C_1-C_4)$alkyl hypochlorite in a step (a) reaction-inert solvent to form a first intermediate, 3-chloro-1,1-di(p-fluorophenyl)urea, in situ;

(b) rearranging the intermediate 3-chlorourea by the action of an alkali metal $(C_1-C_3)$alkoxide in a step (b) reaction-inert solvent to form a second intermediate, 2-$(C_1-C_3)$carbalkoxy-1,1-di(p-fluorophenyl)hydrazine, in situ;

(c) hydrolyzing and decarboxylating the intermediate carbalkoxyhydrazine with water in the presence of base in a step (c) reaction-inert solvent to form a third intermediate, 1,1-di(p-fluorophenyl)hydrazine, in situ;

(d) condensing the intermediate 1,1-di(p-fluorophenyl)hydrazine with N-carbobenzoxy-4-piperidone in the presence of a strong acid in a step (d) reaction-inert solvent, and recovering said 2-carbobenzoxy-8-fluoro-5-(p-fluorophenyl)-2,3,4,5-tetrahydro-1H-pyrido[4,3-b]indole.

2. A process of claim 1 wherein the $(C_1-C_4)$alkyl hypochlorite is t-butyl hypochlorite, the alkali metal alkoxide is sodium methoxide and the second intermediate is 2-carbomethoxy-1,1-(p-diphenyl)hydrazine, and the strong acid is hydrochloric acid.

3. A process of claim 1 or 2 which further comprises hydrogenation of said 2-carbobenzoxy-8-fluoro-5-(p-fluorophenyl)-2,3,4,5-tetrahydro-1H-pyrido[4,3-b]-indole over a noble metal catalyst in the presence of substantially one molar equivalent of 2-(p-fluorophenyl)-5-hydroxytetrahydrofuran, at 0-75°C. in a reaction-inert solvent, to form flutroline.

4. A process of claim 3 wherein the noble metal is palladium and the hydrogen pressure is 2 to 8 atmospheres.

5. A process of claim 1 or 2 which further comprises hydrogenation of said 2-carbobenzoxy-8-fluoro-5-(p-fluorophenyl)-2,3,4,5-tetrahydro-1H-pyrido[4,3-b]indole over a noble metal catalyst at 0-75°C. to form 8-fluoro-5-(p-fluorophenyl)-2,3,4,5-tetrahydro-1H-pyrido[4,3-b]indole.

6. A process of claim 5 wherein the noble metal is palladium and the pressure of hydrogen is 2-8 atmospheres.

7. A process of claim 5 or 6 which further comprises the preparation of flutroline by contacting said 8-fluoro-5-(p-fluorophenyl)-2,3,4,5-tetrahydro-1H-pyrido[4,3-b]indole with at least an equimolar quantity of 2-(p-fluorophenyl)-5-hydroxytetrahydrofuran in a reaction inert solvent in the presence of a reducing agent.

8. A process of claim 7 wherein said reducing agent is sodium cyanoborohydride or hydrogen and a catalytically effective amount of a noble metal catalyst.

9. A process of claim 5 or 6 which further comprises the preparation of flutroline by condensing said 8-fluoro-5-(p-fluorophenyl)-2,3,4,5-tetrahydro-1H-pyrido[4,3-b]indole in a reaction-inert polar organic solvent with at least an equimolar amount of an acetylenic compound of the formula

$$HC\equiv CCHOH(p\text{-}FC_6H_4)$$

and an equimolar amount of formaldehyde, with respect to said acetylenic compound, in the presence of a catalytically effective amount of cuprous chloride

or cuprous bromide; and thereafter hydrogenating the resulting condensation product in a reaction-inert organic solvent at about 15-75°C. and at a hydrogen pressure up to about 75 psig in the presence of a catalytically effective amount of a noble metal catalyst.

10. A process of claim 5 or 6 which further comprises the preparation of the 8-fluoro-5-(p-fluorophenyl)-2-[4-(p-fluorophenyl)-4-hydroxybutyryl]-2,3,4,5-tetrahydro-1H-pyrido[4,3-b]indole by reaction of said 8-fluoro-5-(p-fluorophenyl)-2,3,4,5-tetrahydro-1H-pyrido[4,3-b]indole with 4-(p-fluorophenyl)-tetrahydro-2-furanone at 65-125°C. in a reaction inert solvent.

11. A process of claim 10 which further comprises the preparation of flutroline by reduction of said 8-fluoro-5-(p-fluorophenyl)-2-[4-(p-fluorophenyl)-4-hydroxybutyryl]-2,3,4,5-tetrahydro-1H-pyrido[4,3-b]-indole with sodium bis-(2-methoxyethoxy)aluminum hydride at 80-110°C in a reaction-inert solvent.

12. A process for preparing flutroline which comprises hydrogenation of 2-carbobenzoxy-8-fluoro-5-(p-fluorophenyl)-2,3,4,5-tetrahydro-1H-pyrido[4,3-b]-indole over a noble metal catalyst in the presence of substantially one molar equivalent of 2-(p-fluorophenyl)-5-hydroxytetrahydrofuran, at 0-75°C. in a reaction-inert solvent.

13. A process of claim 12 where the noble metal is palladium and the hydrogen pressure is 2-8 atmospheres.

14. A process for the preparation of flutroline which comprises contacting 8-fluoro-5-(p-fluorophenyl)-2,3,4,5-tetrahydro-1H-pyrido[4,3-b]indole with at least an equimolar quantity of 2-(p-fluorophenyl)-5-hydroxytetrahydrofuran in a reaction inert solvent in the presence of a reducing agent.

15. A process of claim 14 wherein said reducing agent is sodium cyanoborohydride or hydrogen and a catalytically effective amount of a noble metal catalyst.

16. A process for the preparation of flutroline which comprises condensing 8-fluoro-5-(p-fluorophenyl)-2,3,4,5-tetrahydro-1H-pyrido[4,3-b]indole in a reaction inert polar organic solvent with at least an equimolar amount of an acetylenic compound of the formula

$$HC{\equiv}CCHOH(pFC_6H_4)$$

and an equimolar amount of formaldehyde, with respect to said acetylenic compound, in the presence of a catalytically effective amount of cuprous chloride or cuprous bromide; and thereafter hydrogenating the resulting condensation product in a reaction inert organic solvent at about 15-75°C. and at a hydrogen pressure up to about 75 psig in the presence of a catalytically effective amount of a noble metal catalyst.

17. A process for the preparation of flutroline which comprises the reduction of 8-fluoro-5-(p-fluorophenyl)-2-[4-(p-fluorophenyl)-4-hydroxybutyryl]-2,3,4,5-tetrahydro-1H-pyrido[4,3-b]indole with sodium bis-(2-methoxyethoxy)aluminum hydride at 80-110°C in a reaction-inert solvent.

18. A process of claim 17 which further comprises the preparation of the 8-fluoro-5-(p-fluorophenyl)-2-[4-(p-fluorophenyl)-4-hydroxybutyryl]-2,3,4,5-tetrahydro-1H-pyrido[4,3-b]indole by reaction of 8-fluoro-5-(p-fluorophenyl)-2,3,4,5-tetrahydro-1H-pyrido[4,3-b]indole with 4-(p-fluorophenyl)tetrahydro-2-furanone at 65-125°C in a reaction inert solvent.